# EUROPÄISCHE PATENTSCHRIFT

(11) **EP 0 802 924 B1**
(45) Veröffentlichungstag und Bekanntmachung des Hinweises auf die Patenterteilung: **30.12.1998**
(21) Anmeldenummer: 95920061.9
(22) Anmeldetag: 19.05.1995
(51) Int. Cl.: C07K 16/28, A61K 39/395, A61K 47/48

(54) **ARZNEIMITTEL FÜR DIE VERLÄNGERTE IMMUNSUPPRESSION UND TUMORZELLELIMINATION**
MEDICAMENT FOR THE PROLONGED IMMUNOSUPPRESSION AND ELIMINATION OF TUMOUR CELLS
MEDICAMENT POUR L'IMMUNOSUPPRESSION ET ELIMINATION DES CELLULES TUMEUREUSES PROLONGEE

(30) Priorität: 18.06.1994 DE 4421391
(43) Veröffentlichungstag der Anmeldung: 29.10.1997
(73) Patentinhaber: GSF - Forschungszentrum für Umwelt und Gesundheit GmbH, 85764 Oberschleissheim (DE)
(72) Erfinder: THIERFELDER, Stefan, D-82229 Eichenau (DE)
(74) Vertreter: VOSSIUS & PARTNER
(86) Internationale Anmeldenummer: EP9501898
(87) Internationale Veröffentlichungsnummer: WO9535321

(56) Entgegenhaltungen:
- EP-A- 0 336 379
- XIVTH INTERNATIONAL CONGRESS OF THE TRANSPLANTATION SOCIETY, PARIS, FRANCE, AUGUST 16-21, 1992. TRANSPLANT PROC 25 (1 BOOK 1-2). 1993. 842-843. CODEN: TRPPA8 ISSN: 0041-1345, KREMMER E ET AL 'AN ALLOGENEIC IMMUNOSUPPRESSIVE MONOCLONAL ANTI- T CELL ANTIBODY CAN ELICIT NEUTRALIZING ANTI - ANTIBODIES IN MICE.'
- EUR. J. IMMUNOL. (1994), 24(10), 2323-8 CODEN: EJIMAF;ISSN: 0014-2980, 1994 MYSLIWIETZ, JOSEF ET AL 'Immunological approach to inhibit formation of anti - antibodies to allo- and xenogeneic anti- T cell immunoglobulin'
- EUR. J. IMMUNOL. (1993), 23(5), 1017-22 CODEN: EJIMAF;ISSN: 0014-2980, 1993 KREMMER, ELISABETH ET AL 'Murine anti-mouse T cell monoclonal antibodies elicit anti - antibodies in mice: Intra-species immunization model for estimating potential patient sensitization against humanized anti- T cell antibodies' in der Anmeldung erwähnt
- JOURNAL OF IMMUNOLOGY, Bd. 140, Nr. 11, 1.Juni 1988 BALTIMORE US, Seiten 3766-3772, HIRSCH R. ET AL. 'Effects of in vivo administration of anti-T3 monoclonal antibody on T-cell function in mice' in der Anmeldung erwähnt

## Beschreibung

Die Erfindung betrifft Arzneimittel für die verlängerte Immunsuppression und Tumorzellelimination.

Eine Transplantatabstoßung wird mit Immunsuppressiva, z. B. monoklonalen, immunsuppressiven, aus Mäusen, Ratten oder Goldhamstern generierten Antikörpern gegen menschliche T-Lymphozyten behandelt. Ihre Wirkung wird jedoch begrenzt durch eine Immunreaktion, die der Patient gegen die von einer anderen Tierspezies stammenden Antikörperproteine entwickelt. Dabei entstehen sog. Anti-antikörper, die die immunsuppressive Wirkung der injizierten Antikörper hemmen. So werden derzeit z. B. Abstoßungskrisen bei Patienten mit Nierentransplantaten meist nur mit einer einmaligen Antikörpertherapie behandelt. Bei erneuter Abstoßungskrise wird diese Behandlung wegen Anti-antikörperbildung im allgemeinen nicht wiederholt.

Eine klinische Therapie der Wahl zur Verlängerung der immunsuppressiven Antikörperwirkung unter Vermeidung von Anti-antikörperbildung gibt es bisher nicht. Eine Wiederbehandlung mit einem anderen monoklonalen Antikörper führt ebenfalls - u. U. sogar beschleunigt - zu Anti-antikörperbildung [1]. Patienten entwickelten auch Anti-antikörper gegen immunsuppressive Antikörper, die mit gentechnischen Methoden humanisiert d. h. der "Primatenspezies" des Patienten weitgehend angeglichen, "spezies-adaptiert" worden waren [2].

Experimentell, im Mausmodell, wurde eine deutliche, sogar als Toleranzinduktion bewertete Verlängerung der Überlebenszeit von Hauttransplantaten mitgeteilt. Sie wurde beobachtet nach Injektion von hohen Dosen eines gegen Maus T(L3T4+Lyt-2)-Zellen gerichteten Rattenantikörperpaars gefolgt von einem zweiten, spezies-gleichen Antikörperpaar gleicher Zellbindungsspezifität, das sich jedoch vom ersten durch seine geringe Elimination von T Zellen aus dem Blutkreislauf der Maus unterschied (="non-depleting" nicht depletierendes d.h. eliminierendes Antikörperpaar). Das beschriebene Wirkungsprinzip basiert im Gegensatz zur Erfindung nicht auf einer kombinierten Therapie von mindestens 2 Antikörpern mit speziesdifferenter Fc-Region [3].

Verlängerte Überlebenszeit von Hauttransplantaten und fehlende Anti-antikörperbildung wurde weiterhin mitgeteilt nach Injektion eines Ratte anti-Maus T(L3T4 = CD4+ Lymphozytensubpopulation)-Zellantikörpers mit folgender Injektion von (Fab')2 Fragmenten und unfragmentierten monoklonalen Hamster anti-Maus T(CD3)-Antikörpern[4]. Auch hier wird das beschriebene Wirkungsprinzip im Gegensatz zur Erfindung nicht auf eine kombinierte Therapie von zwei, gegen alle T-Zellen gerichteten Antikörper mit speziesdifferenter Fc-Region zurückgeführt sondern auf die mit dem ersten Antikörper erzielte Suppression der CD4+ T-Lymphozytensubpopulation [5], diese reicht jedoch nicht aus.

Dauerhafte Toleranz von Hauttransplantaten kann mit einer anderen, allerdings risikobelasteten Methode in bestrahlten Mäusen nach Transplantation von Knochenmark des Hauttransplantatspenders und unter dem Schutz von Anti-T Zellantikörpern erzielt werden [6].

Es gibt bisher keine Therapie der Wahl bei konventionellen poly- oder monoklonalen immunsuppressiven Antikörpern, eine Anti-antikörperbildung im Patienten sicher zu verhindern. Auch die ersten klinischen Erfahrungen mit neuerdings gentechnisch humanisierten Antikörpern zeigen, daß Anti-antikörper gebildet werden können [2] ähnlich wie das mit murinen immunsuppressiven anti-Maus T-Zellantikörpern gezeigt wurde [7].

Eine Kombination von immunsuppressiver Antikörperbehandlung mit Chemotherapie, z. B. Cyclophosphamid oder Busulfan, birgt das Risiko von Nebenwirkungen besonders auf die Blutbildung aber auch auf das transplantierte Gewebe aufgrund der mangelnden Zellspezifität von Chemotherapeutika [3, 8]

Aufgabe der Erfindung ist es, Arzneimittel enthaltend Antikörper für eine klinische Therapie zur Verlängerung der immunsuppressiven Antikörperwirkung unter Vermeidung von Anti-antikörperbildung zur Verfügung zu stellen.

Gelöst wird diese Aufgabe durch die Merkmale des Patentanspruchs 1.

Die Unteransprüche beschreiben vorteilhafte Ausgestaltungen der Erfindung.

Jede der beiden Gruppen A, B von Antikörpern kann auch jeweils nur aus einer Antikörperart oder mehreren Antikörperarten bestehen. Die Gruppen A, B können also monoklonal oder polyklonal sein. Die hier dargestellte sequentielle Behandlung mit molekularbiologisch partiell oder voll humanisierten und nicht- humanisierten Anti-T Zellantikörpern - oder mit mindestens zwei Anti-T Zellantikörpern aus unterschiedlichen Spezies generiert - führt zur Verlängerung von Immunsuppression und Tumorzellsuppression. Sie ist ein tierexperimentell erprobtes, bisher nicht beschriebenes Behandlungsprinzip.

Das Neue, von anderen Autoren bisher nicht erkannte bzw. mitgeteilte Therapieprinzip der Erfindung ist immunologisch überhaupt nicht naheliegend. Üblicherweise suchen Immunbiologen und Mediziner eine Minderung der Immunogenität von anti-T Zellantikörpern (die die Anti-antikörperbildung hervorruft) in deren möglichst weitgehenden Angleichung an die Antikörper immunglobulinstrukturen des Patienten, damit dieser sie eher toleriert, z. B. durch gentechnische Humanisierung der aus Mäusen generierten monoklonalen immunsuppressiven Antikörper. Diese Angleichung ist aber im Prinzip letztlich nicht vollständig möglich und Ursache für Anti-antikörperbildung, weil die T-zellbindende (V) Region des immunusuppressiven Antikörpers so variabel ist, daß der Immunapparat des Patienten eben doch Anti-antikörper gegen sie bilden kann.

Die Erfindung basiert vielmehr auf einer gegenteiligen Erfahrung, nämlich auf Anti-antikörperunterdrückung durch Herstellung einer hochgradigen Speziesdifferenz von anti-T Zell Antikörpern, wovon einer oder beide, für sich allein appliziert, potentiell immunogen im Antikörperempfänger sein darf.

Es wurde erkannt,
a) daß die Überlebenszeit von Hauttransplantaten grundsätzlich verlängert wurde nicht dadurch, daß Mäusen zwei verschiedene Maus anti-Maus T-Zellantikörper (oder zwei Ratte anti-Maus T-Zellantikörper) hintereinander appliziert wurden sondern daß zwei zu einander, nicht notwendigerweise aber zum Antikörperempfänger spezies-differente Antikörper zu deutlich verlängerter Immunsuppression führen
b) sogar zwei Antikörper auch dann wirksam sind, wenn sie sich so weit unterscheiden wie Mensch und Maus.

Diese Überlegungen, Ergebnisse und Antikörperkombinationen definieren ein Therapiemodell, das u. a. den Vorteil besitzt, daß es klinisch sofort erprobt werden kann und den Patienten keinen zusätzlichen Behandlungsrisiken aussetzt. Eine Verlängerung von Immunsuppression dürfte nicht nur Abstoßungskrisen von Organtransplantaten und Immunkomplikationen bei Knochenmarktransplantation erfolgreicher therapieren sondern durch prophylaktische Behandlung überhaupt verhindern helfen. Auch Autoimmunkrankheiten, chronische Krankheiten des rheumatischen Formenkreises aber auch einzelnen Tumorerkrankungen dürften sich neue therapeutische Perspektiven eröffnen. So zeigen eigene Untersuchungen zur Unterdrückung von murinen oder menschlichen, auf Mäuse transplantierte T Zell-Leukämien im Mausmodell eine mittels Antikörperinjektion verlängerte Überlebenszeit. Nach T-Zelldepletion können bei chimären, d. h. mit Knochenmark transplantierten und an Leukämie erkrankten Mäusen fremde immunkompetente Zellen eingeschleust werden, die im Empfänger die neoplastischen Zellen attackieren. Die unter b) beschriebene Toleranz induktion gegenüber artfremden Serumeiweiß eröffnet darüber hinaus eine von Überempfindlichkeitsreaktionen befreite passive Impfung mit Antikörpern einer fremden Spezies z. B. bei Tetanus.

Im Hauttransplantationsmodell der Maus konnte gezeigt werden, daß ein gentechnisch humanisierter monoklonaler, immunsuppressiver Antikörper gegen T-Lymphozyten der Maus eine um das Vielfache verlängerte Überlebenszeit von Transplantaten erzielt, wenn seiner Applikation oder mehrere Injektionen eines monoklonalen immunsuppressiven Mausantikörpers vorgeschaltet wurde. Für sich allein mußten die vorgeschalteten Antikörperinjektionen nicht immunsuppressiv im Sinn einer Transplantatverlängerung sein. Wie sich zeigte, hatte diese Antikörpertherapie eine komplette Toleranz gegen artfremdem, humanem Serumeiweiß induziert, die auch fünf Monate nach Beendigung der immunsuppressiven Therapie anhielt. Die unerwartete Verlängerung der immunsuppressiven Wirkung war infolgedessen von einem Ausbleiben der Anti-antikörperbildung in den behandelten Mäusen aufgrund ihrer Toleranz des artfremden Antikörperimmunglobulins begleitet. Das diesem Phänomen zugrunde liegende Wirkungsprinzip wird von uns analysiert besonders im Hinblick auf spezies-bedingte Unterschiede in der Fc-Region der kombinierten Antikörper. Es zeigte sich auch wirksam, wenn nicht molekularbiologisch veränderte Anti-T Zellantikörper benutzt wurden vorausgesetzt sie waren zueinander speziesdifferent.

Antikörper haben einen sog. variablen, die Antikörperbindungsstelle einschließenden Bereich und einen sog. konstanten, Antikörpereffektorfunktionen (z. B. Elimination von mit Antikörpern besetzten Körperzellen aus dem Kreislauf) vermittelnden Fc Bereich (Fc-Region), der auf den sog. konstanten Teilen der schweren Ketten des Antikörpers lokalisiert ist. So können sich zwei Antikörper gleichen hinsichtlich ihrer Spezifität z.B. menschliche T-Lymphozyten zu binden. Solche Antikörper mit gleicher Zellbindungsspezifität können sich jedoch unterscheiden in ihrer Fc-Region aufgrund ihrer Herkunft aus verschiedenen normalen oder molekularbiologisch manipulierten Tierspezies. Sie können auch in vitro in der Fc-Region mit molekularbiologischen Methoden verändert werden nach Generierung von antikörpersezernierenden Zellen (z. B. Hybridome oder Hybridhybridome), so daß es zu einem Grad an Unterschiedlichkeit kommt, wie er zwischen Mensch und Nager besteht und in der Erfindung beschrieben wird.

Die Erfindung wird im folgenden anhand dreier Beispiele mit Hilfe der Figuren näher erläutert.

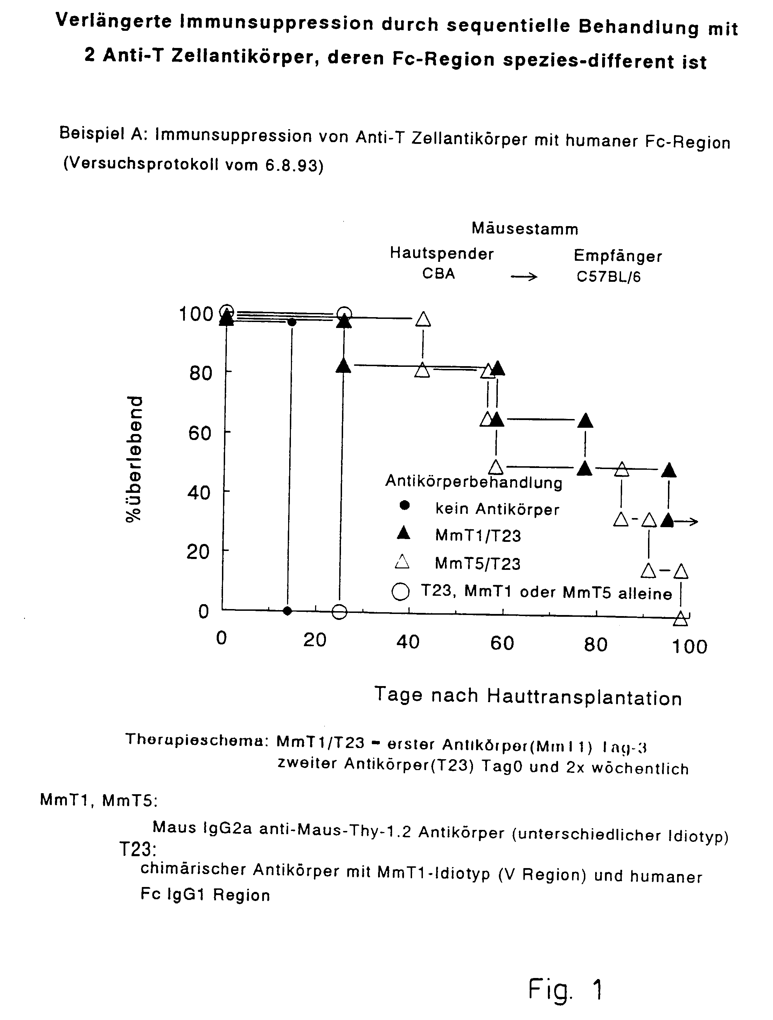

Dabei zeigen die Fig. 1, 2 und 3 jeweils ein schematisertes Versuchsprotokoll der Beispiele A, B und C.

Fig. 1, (Beispiel A) zeigt eine um etwa zehnfach verlängerte Immunsuppression gemessen in einem Nagerhauttransplantationsmodell von maximaler Histoinkompatibilität. Sie wurde durch kombinierte, Fc-Region- imkompatible Antikörperbehandlung erzielt. Bei der MmT1/T23 Antikörperbehandlung wurde zunächst eine Injektion eines Maus anti-Maus T Zellantikörpers (MmT1[7]) appliziert, danach folgte zweimal wöchentlich der T23 Antikörper, der sich von MmT1 durch gentechnisch erzielten Austausch der murinen gegen eine humane IgG1 Fc-Region unterscheidet. Eine Einzeldosis von MmT1 verlängerte die (mittlere) Hautüberlebenszeit nicht. T23 allein, wöchentlich 2x appliziert verlängerte sie um neun Tage von 16 auf 24, MmT1(Erstdosis) gefolgt von T23(wöchentlich 2x appliziert) verlängerte sie auf über 90 Tage. Eine ähnlich gesteigerte Immunsuppression wurde auch nach Austauch von MmT1 gegen MmT5 [7] erzielt, der sich von MmT1 nicht in der T-Zellspezifität unterscheidet sondern in der Feinstruktur der Antikörperbindungsstelle (Idiotyp). Daraus ergibt sich die Schlußfolgerung, daß bei der kombinierten Fc-Region-inkompatiblen Antikörpertherapie die Gleichheit oder Differenz der Antikörperbindungsstelle keine Voraussetzung für das Wirkungsprinzip darstellt sondern die speziesbedingte Differenz ihrer die Fc-Regionen. einschießenden schweren Ketten.

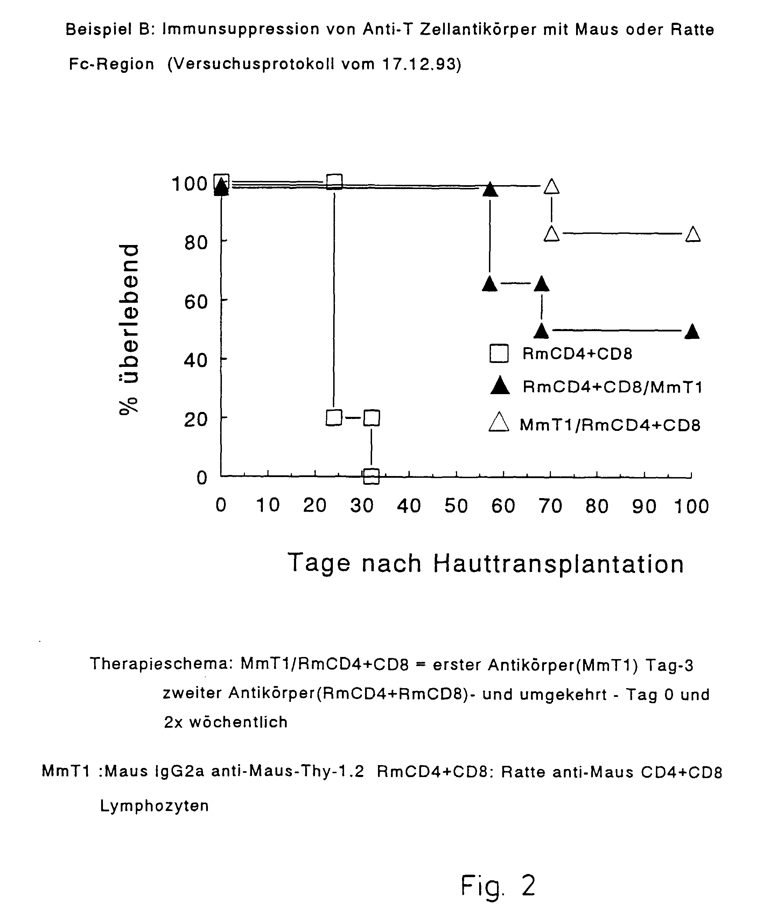

Fig. 2 (Beispiel B) zeigt, daß Ratte anti-Maus T-Zellantikörper mit Thy-1 Spezifität [9] und auch besonders klinikrelevante Antikörperspezifitäten wie anti-CD4 und anti-CD8 (2 T-Zellsubpopulation, die zusammen alle T-Zellen binden) durch Vorinjektion einer Dosis MmT1 in der MmT1/RmCD4+CD8 Kombination die mittlere Überlebenszeit von Hauttransplantaten ebenfalls verlängern. Darüber hinaus wird gezeigt, daß die Umkehrung der kombinierten Antikörperbehandlung in der RmCD4+CD8/MmT1 Kombination auch zur verlängerten Immunsuppression von MmT1 führt, da auch hier die Vorbedingung der speziesbedingten Differenz der Fc-Region erfüllt ist. Da auch anti-CD4+CD8 als Erstantikörper wirksam sind, schließt dies eine auf MmT1 beschränkte Wirkung des vorinjizierten Erstantikörpers aus. Vielmehr gilt immer wieder die Voraussetzung für die syngeristische Antikörperwirkung ihre speziesbedingten Fc-Region Differenzen (abgesehen von der zeitungleichen Applikation von mindestens zwei Antikörpern). Ein Überleben von Hauttransplantaten bei anhaltender Antikörpertherapie war schließlich dauerhaft, wenn dem zweiten Antikörper noch weitere T-Zell-depletierende und/oder T-zellrezeptor-modulierende (anti-CD3)Antikörper beigegeben wurden.

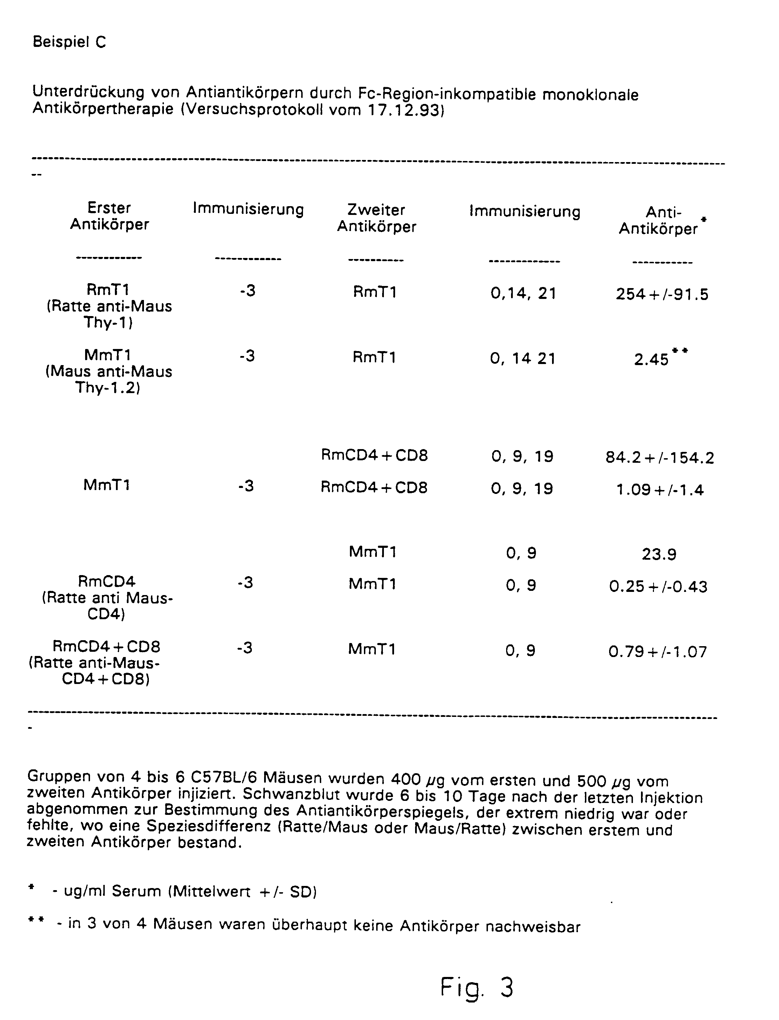

Fig. 3 (Beispiel C) belegt am Beispiel B, daß es bei der kombinierten Ratte/Maus oder Maus/Ratte Fc-Region inkompatiblen Antikörperbehandlung zu hochgradiger Suppession oder gänzlichem Fehlen von Antiantikörperbildung kommt. Gleiches trifft auch auf Beispiel A zu.

Anti-antikörper entstehen auch bei Behandlung mit polyklonalen Antikörpern wie sie nach Immunisation von z. B. Kaninchen-, Ratten- oder Pferdelymphozyten entstehen; auch hier zeigt sich, daß eine Speziesdifferenz (Beispiel Kaninchen/Ratte) von polyklonalen Antikörpern zur verlängerten Immunsuppression in Mäusen führt, ebenso mit sog. bispezifischen Antikörpern, d. h. Antikörpern mit zwei unterschiedlichen Bindungsstellen oder mit chemisch durch Einführung einer niedermolekularen Verbindung (z. B. DNP, TNP Haptene) veränderten Anti-T Zellantikörpern oder mit gentechnisch, z. 3. in Bakterien hergestellte Antikörper und Antikörperfragmente. Auch hier können sich sequentiell injizierte Anti-T Zellantikörper in der Anti-antikörperbildung gegen speziesdifferente polyklonale oder bispezifische oder chemisch oder molekularbiologisch veränderte Antikörper neutralisieren. Voraussetzung ist immer wieder eine starke Differenz der sequentiell applizierten Antikörper oder Antikörpergruppen wie sie sich entweder aus der Speziesdifferenz ergibt oder aus der Einführung (Konjugation) von chemischen Verbindungen.

Schließlich können unerwünschte Immunreaktionen auch bei passiver Immunisation mit Antikörpern in eiweis-überempfindlichen oder praesensibilisierten Patienten auftreten, wo eine präventive Behandlung durch Kombinierte Fc-Region-inkompatible Antikörpertherapie die Antiantikörperbildung verhindert.

### Literaturnachweis:

[1]
   Chatenoud, L., Transpl. Proc. 1993. 25, 2, Suppl.1: 68.
[2]
   Isaacs, J.D., Watts, R.A., Hazleman, B.L., Hale, G., Keogan, M.T., Cobbold, S.P. and Waldmann, H., Lancet. 1992. 340: 748.
[3]
   Cobbold, S.P., Martin, G. and Waldmann, H., Eur. J. Immunol 1990. 20: 2747.
[4]
   Hirsch, R., Chatenoud, L., Gress, R.E., Sachs, D.H., Bach, J.F. and Bluestone, J.A., Transplantation 1989. 47: 853.
[5]
   Hirsch, R., Eckhaus, M., Auchincloss, H.J., Sachs, D.H. and Bluestone, J.A., J. Immunol 1988. 140: 3766.
[6]
   Thierfelder, S., Kummer, U., Schuh, R. and Mysliwietz, J., Blood 1986. 68: 818.
[7]
   Kremmer, E., Mysliwietz, J., Lederer, R. and Thierfelder, S., Eur. J Immunol 1993. 23: 1017.
[8]
   Leong, L.Y.W., Qin, S., Cobbold, S.P. and Waldmann, H., Eur. J. Immunol. 1992. 22: 2825.
[9]
   Kummer, U., Thierfelder, S., Hoffmann-Fezer, G. and Schuh, R., J. Immunol 1987. 138: 4069.

## Patentansprüche

1. Arzneimittel für die verlängerte Immunsuppession und Tumorzellelimination, getrennt enthaltend mindestens zwei verschiedene Gruppen Antikörper A, B gegen T-Zellen, wobei mindestens eine Antikörperart aus der Gruppe B in den konstanten Teilen der schweren Kette von mindestens einer Antikörperart der Gruppe A speziesdifferent und damit einer unterschiedlichen Tierart zuzuordnen ist und wobei die Gruppe A, welche als erste einmal oder mehrmals appliziert wird, T-Zelleliminierend wirkt, während die weitere Gruppe B (welche in zeitlichem Abstand appliziert wird) T-Zelleliminierend und/oder T-Zellantigen-modulierend wirkt.

2. Arzneimittel nach Anspruch 1, dadurch gekennzeichnet, daß eine Antikörperart aus der Gruppe B bispezifisch ist.

3. Arzneimittel nach Anspruch 1 oder 2, dadurch gekennzeichnet, daß eine Tierart der Mensch ist.

4. Arzneimittel nach einem der Ansprüche 1 bis 3, dadurch gekennzeichnet, daß eine Antikörperart aus einer der Gruppen A, B in seinen konstanten Teilen der schweren Ketten gentechnisch gegenüber einer Antikörperart der anderen Gruppe verändert ist.

5. Arzneimittel nach Anspruch 4, dadurch gekennzeichnet, daß der Antikörper humanisiert ist.

6. Arzneimittel nach einem der Ansprüche 1 bis 5, dadurch gekennzeichnet, daß eine Antikörperart aus einer der Gruppen A, B in seinen konstanten Teilen der schweren Ketten durch Einführung von chemischen Verbindungen gegenüber einer Antikörperart der anderen Gruppe verändert (z.B. DNP-haptenisiert) ist.

7. Arzneimittel nach einem der Ansprüche 1 bis 6, dadurch gekennzeichnet, daß mindestens eine der Gruppen A, B polyclonale Antikörper umfaßt.

8. Arzneimittel nach einem der Ansprüche 1 bis 6, dadurch gekennzeichnet, daß mindestens eine der Gruppen A, B monoclonale Antikörper umfaßt.

9. Verwendung von mindestens zwei verschiedenen Gruppen Antikörpern A, B gegen T-Zellen, wobei mindestens eine Antikörperart aus der Gruppe B in den konstanten Teilen der schweren Kette von mindestens einer Antikörperart der Gruppe A speziesdifferent ist, und wobei die Gruppe A T-Zelleliminierend wirkt, während die weitere Gruppe B T-Zelleliminierend und/oder T-Zellantigen-modulierend wirkt zur Herstellung eines Arzneimittels nach einem der Ansprüche 1 bis 8.

10. Verwendung von mindestens zwei verschiedenen Gruppen Antikörpern A, B gegen T-Zellen, wobei mindestens eine Antikörperart aus der Gruppe B in den konstanten Teilen der schweren Kette von mindestens einer Antikörperart der Gruppe A speziesdifferent ist, und wobei die Gruppe A T-Zelleliminierend wirkt, während die weitere Gruppe B T-Zelleliminierend und/oder T-Zellantigen-modulierend wirkt, zur Herstellung eines Arzneimittels zur verlängerten Immunsuppression, wobei zunächst die Antikörper der Gruppe A ein- oder mehrmals appliziert werden und sodann in zeitlichem Abstand dazu die Antikörper der Gruppe B appliziert werden.

11. Verwendung von mindestens zwei verschiedenen Gruppen Antikörpern A, B gegen T-Zellen, wobei mindestens eine Antikörperart aus der Gruppe B in den konstanten Teilen der schweren Kette von mindestens einer Antikörperart der Gruppe A speziesdifferent ist, und wobei die Gruppe A T-Zelleliminierend wirkt, während die weitere Gruppe B T-Zelleliminierend und/oder T-Zellantigen-modulierend wirkt, zur Herstellung eines Arzneimittels zur Tumorzellelimination, wobei zunächst die Antikörper der Gruppe A ein- oder mehrmals appliziert werden und sodann in zeitlichem Abstand dazu die Antikörper der Gruppe B appliziert werden.

## Claims

1. A pharmaceutical for a prolonged immunosuppression and tumor cell elimination, separately containing at least two different groups of antibodies A, B against T cells, wherein at least one antibody type of group B is species different from at least one antibody type of group A in the constant regions of their heavy chains and thus belongs to a different animal species and wherein group A, which is first applied once or several times, has a T-cell eliminating effect, whereas the other group B (which is applied at a different time) has a T-cell eliminating and/or T-cell antigen modulating effect.

2. The pharmaceutical according to Claim 1, characterized in that one antibody type of group B is bispecific.

3. The pharmaceutical according to Claim 1 or 2, characterized in that one animal species is human.

4. The pharmaceutical according to one of Claims 1 to 3, characterized in that one antibody type of one of groups A, B is modified in the constant regions of its heavy chains vis-à-vis one antibody type of the other group by genetic engineering.

5. The pharmaceutical according to Claim 4, characterized in that the antibody is humanized.

6. The pharmaceutical according to any one of Claims 1 to 5, characterized in that one antibody type of one of groups A, B is modified in its constant heavy chains by introduction of chemical compounds vis-à-vis one antibody type of the other group (e.g. DNP-haptenized).

7. The pharmaceutical according to any one of Claims 1 to 6, characterized in that at least one of groups A, B comprises polyclonal antibodies.

8. The pharmaceutical according to any one of Claims 1 to 6, characterized in that at least one of groups A, B comprises monoclonal antibodies.

9. The use of at least two different groups of antibodies A, B against T cells, wherein at least one antibody type of group B is species different from at least one antibody type of group A in the constant regions of their heavy chains, and wherein group A has a T-cell eliminating effect, whereas the other group B has a T-cell eliminating and/or T-cell antigen modulating effect for preparing a pharmaceutical of any of Claims 1 to 8.

10. The use of at least two different groups of antibodies A, B against T cells, wherein at least one antibody type of group B is species different from at least one antibody type of group A in the constant regions of their heavy chains, and wherein group A has a T-cell eliminating effect, whereas the other group B has a T-cell eliminating and/or T-cell antigen modulating effect for preparing a pharmaceutical for prolonged immunosuppression, wherein first the antibodies of group A are applied once or several times and then the antibodies of group B are applied in addition after a certain amount of time.

11. The use of at least two different groups of antibodies A, B against T cells, wherein at least one antibody type of group B is species different from at least one antibody type of group A in the constant regions of their heavy chains, and wherein group A has a T-cell eliminating effect, whereas the other group B has a T-cell eliminating and/or T-cell antigen modulating effect for preparing a pharmaceutical for eliminating tumor cells, wherein first the antibodies of group A are applied once or several times and then the antibodies of group B are applied in addition after a certain amount of time.

## Revendications

1. Médicament pour prolonger la suppression immunitaire et l'élimination des cellules tumorales, contenant séparément au moins deux groupes différents d'anticorps A, B contre les cellules T, où au moins un type d'anticorps du groupe B présente, dans les régions constantes de la chaîne lourde, une différence d'espèce avec au moins un type d'anticorps du groupe A et doit donc être affecté à une espèce animale différente, et où le groupe A, qui en premier est administré en une ou plusieurs fois, à un effet d'élimination des cellules T, tandis que l'autre groupe B (qui est administré à un autre instant) a un effet d'élimination des cellules T et/ou de modulation de l'antigène des cellules T.

2. Médicament selon la revendication 1, caractérisé en ce qu'un type d'anticorps du groupe B est bispécifique.

3. Médicament selon la revendication 1 ou 2, caractérisé en ce qu'une espèce animale est l'homme.

4. Médicament selon l'une des revendications 1 à 3, caractérisé en ce qu'un type d'anticorps de l'un des groupes A, B subit, dans ses régions constantes de la chaîne lourde, une modification génétique par rapport à un type d'anticorps de l'autre groupe.

5. Médicament selon la revendication 4, caractérisé en ce que l'anticorps est humanisé.

6. Médicament selon l'une des revendications 1 à 5, caractérisé en ce qu'un type d'anticorps des groupes A, B subit, dans ses parties constantes des chaînes lourdes, par introduction de composés chimiques, une modification par rapport à un type d'anticorps de l'autre groupe (par exemple DNP-hapténisé).

7. Médicament selon l'une des revendications 1 à 6, caractérisé en ce qu'au moins l'un des groupes A, B comporte des anticorps polyclonaux.

8. Médicament selon l'une des revendications 1 à 6, caractérisé en ce qu'au moins l'un des groupes A, B comporte des anticorps monoclonaux.

9. Utilisation d'au moins deux groupes différents d'anticorps A, B contre des cellules T, où au moins un type d'anticorps du groupe B présente dans les parties constantes de la chaîne lourde une différence d'espèce avec au moins un type d'anticorps du groupe A, et où le groupe A un effet d'élimination des cellules T, tandis que l'autre groupe B a un effet d'élimination des groupes T et/ou de modulation de l'antigène des cellules T, pour préparer un médicament selon l'une des revendications 1 à 8.

10. Utilisation d'au moins deux groupes différents d'anticorps A, B contre des cellules T, où au moins un type d'anticorps du groupe B présente dans les parties constantes de la chaîne lourde une différence d'espèce avec au moins un type d'anticorps du groupe A, et où le groupe A un effet d'élimination des cellules T, tandis que l'autre groupe B a un effet d'élimination des groupes T et/ou de modulation de l'antigène des cellules T, pour préparer un médicament destiné à prolonger la suppression immunitaire, où on administre d'abord les anticorps du groupe A en une ou plusieurs fois, puis, au bout d'un certain temps, on administre les anticorps du groupe B.

11. Utilisation d'au moins deux groupes différents d'anticorps A, B contre des cellules T, où au moins un type d'anticorps du groupe B présente dans les parties constantes de la chaîne lourde une différence d'espèce avec au moins un type d'anticorps du groupe A, et où le groupe A un effet d'élimination des cellules T, tandis que l'autre groupe B a un effet d'élimination des groupes T et/ou de modulation de l'antigène des cellules T, pour préparer un médicament destiné à l'élimination des cellules tumorales, où on administre d'abord en une ou plusieurs fois les anticorps du groupe A, puis, au bout d'un certain temps, on administre les anticorps du groupe B.
